# EUROPÄISCHE PATENTANMELDUNG

(11) **EP 2 116 225 A1**
(43) Veröffentlichungstag der Anmeldung: **11.11.2009**
(21) Anmeldenummer: 09154598.8
(22) Anmeldetag: 09.03.2009
(51) Int. Cl.: A61K 8/81, A61Q 5/12

(54) **Ausspülhaarspülungen zur Steigerung des Frisurvolumens**

(30) Priorität: 08.05.2008 DE 102008051860
(71) Anmelder: Beiersdorf AG, 20253 Hamburg (DE)
(72) Erfinder: Franck, Kerstin, 25451, Quickborn (DE); Heuer, Björn, 22145, Hamburg (DE); Wächter, Anna, 20251, Hamburg (DE); Mahadeshwar, Anand, 22529, Hamburg (DE)

(57) **Zusammenfassung**

Kosmetische Zubereitungen zur Behandlung keratinischer Fasern mit kationischen Tensiden, enthaltend Polyquaternium-68 und Polymer (I) zusammengesetzt aus 45 bis 49 Gew.-% tert. Butylacrylat, 23 bis 27 Gew.-% Vinylpyrrolidon, 9 bis 11 Gew.-% Dimethylaminopropylmethacrylamid, 13 bis 17 Gew.-% Methacryloyloxyethyldimethylethyl Ammonium Ethylsulfat, 2 bis 4 Gew.-% Methacrylsäure, besonders bevorzugt aus 47 Gew.-% tert. Butylacrylat, 25 Gew.-% Vinylpyrrolidon, 10 Gew.-% Dimethylaminopropylmethacrylamid, 15 Gew.-% Methacryloyloxyethyldimethylethyl Ammonium Ethylsulfat, 3 Gew.-% Methacrylsäure mit einem Molekulargewicht von 80 000 bis 240 000 g/mol.

## Beschreibung

Die vorliegende Erfindung betrifft kosmetische und dermatologische Zubereitungen zur Volumensteigerung keratinhaltiger Fasern durch Ausspülhaarspülungen (rinse-off Haarspülungen) mit bestimmten amphoteren/ kationischen Polymere.

Die Nutzung von Haarspülungen ist insbesondere für Frauen mit trockenem/ strapazierten Haar wichtig, um Kämmbarkeit, Geschmeidigkeit etc. der Haare zu verbessern.

Aber auch Frauen mit feinem Haar nutzen Haarspülungen, haben aber Probleme mit Beschwerungseffekten und fehlendem Volumen, was durch ein zu hohes Maß an Pflegestoffen hervorgerufen werden kann.

Kurzum: es hat bereits Versuche gegeben, Ausspülhaarspülungen mit Volumensteigerung zu finden. Dennoch sind die bekannten Zubereitungen nicht in jeder Hinsicht optimal.

Die Schrift WO 2008031892 A1 bewschreibt ein ampholytisches Copolymer, das einen molaren Überschuss an katiogenen/- kationischen Gruppen gegenüber aniogenen/ anionischen Gruppen aufweist.

Die Schrift WO 2001/062809 beschreibt Kosmetika aus drei bestimmten Copolymeren.

Die Schrift WO 2005/058988 beschreibt bestimmte ampholytische Copolymere, die sich zu Festigungszwecken eignen sollen.

Die Schrift WO 2004/058837 offenbart bestimmte ampholytische Copolymere, kosmetische oder pharmazeutische Mittel, diese enthaltend in einer Kombination mit einer Vielzahl weiterer Komponenten in verschiedenen Produktformen, darunter auch Haarfestiger.

Die Schrift WO 1995/035087 beschreibt ein haarfixierendes, amphoteres Polymer, das Hydroxyethylmethacrylat als Copolymer enthalten kann.

Die Schrift US 4237253 offenbart bestimmte Copolymere auf Acrylbasis und deren Herstellungsverfahren.

Die Schrift EP 0330 174 Patent beschreibt Haarfixiergele mit vernetzten Carboxyvinyl Polymeren, amphoteren Polymeren und 85 bis 99,3% wässriges und/ oder alkoholisches Lösungsmittel.

Die Schrift US 6589539 B1 beschreibt Kosmetikprodukte mit verzweigten und/ oder quervernetzten amphoteren Polymeren, die eine Verbesserung der Entwirrbarkeit (besonders im feuchten Haar) und eine Verbesserung der Haarweichheit mit sich bringt.

Es war eine Aufgabe der vorliegenden Erfindung, den Übelständen des Standes der Technik abzuhelfen und eine Volumensteigerung keratinhaltiger Fasern durch Ausspülhaarspülungen zu erreichen und insbesondere für Frauen mit trockenem/ strapazierten Haar die Kämmbarkeit, Geschmeidigkeit etc. der Haare zu verbessern.

Auch für Frauen mit feinem Haar sollen solche Ausspülhaarspülungen bereitgestellt werden, die keine Beschwerungseffekte hervorrufen.

Überraschend und für den Fachmann nicht vorhersehbar hat sich herausgestellt, dass kosmetische Zubereitungen zur Behandlung keratinischer Fasern mit kationischen Tensiden enthaltend Polyquaternium-68 und Polymer 1, zusammengesetzt aus 23 bis 27 Gew.-% Vinylpyrrolidon, 45 bis 49 Gew.-% *tert*-Butylacrylat, 2 bis 4 Gew.-% Methacrylsäure, 9 bis 11 Gew.-% Dimethylaminopropylmethacrylamid und 13 bis 17 Gew.-% Methacryloyloxyethyldimethylethyl Ammonium Ethylsulfat, mit einem Molekulargewicht von 80 000 bis 240 000 g/mol, mit der Vorgabe das Polymer mit einer geeigneten Säure neutralisiert ist, den Nachteilen des Stand der Technik abhelfen.

Das Polymer 1 kann durch folgende Formel (I) beschrieben werden:

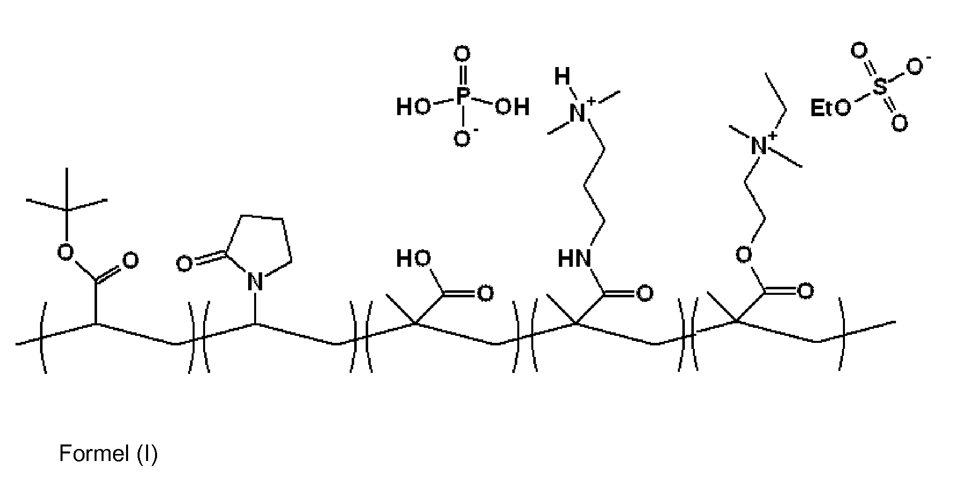

Anteile der Monomere:
47 Gew.-% TBA (tert. Butylacrylat),
25 Gew.-% VP (Vinylpyrrolidon),
10 Gew.-% DMAPMAM (Dimethylaminopropylmethacrylamid),
15 Gew.-% q-DMAEMA (Methacryloyloxyethyldimethylethyl Ammonium Ethylsulfat),
3 Gew.-% MAS (Methacrylsäure).

Ganz besonders bevorzugt ist es, wenn Polyquaternium-68 zusätzlich enthalten ist. Durch die Verwendung einer Kombination aus diesem kationischen Polymer (Polyquaternium-68, BASF, quarternäres Ammoniumsalz des Polymers aus Vinylpyrrolidon, Methacrylamid, Vinylimidazol und 3-Methyl-1-Vinylimidazoliummethylsulfat) und einem amphoteren Polymer nach Formel (I) (experimentelles Polymer 1, BASF) ist es möglich, das Haarvolumen aus einer Spülungsapplikation zu steigern.

Hierbei wichtig zu erwähnen, ist, dass sich das Volumen nur durch die Kombination der beiden Rohstoffe steigern lässt, nicht aber durch den Einsatz der Einzelrohstoffe.

Unter keratinischen Fasern versteht man Haare und Haarprodukte wie menschliche Haare, ganz gleich an welchem Körperteil befindlich, insbesondere das natürliche eigene Kopfhaar, aber auch Toupets, Haare an Haustieren (Katzen, Schafe, Hunde, gezähmte Nagetiere) und Haarprodukte wie etwa Pelze, Wollerzeugnisse (Teppiche, Kleidung).

Die Vergrößerung des Frisurvolumens ist eine Kombination aus visuellem und taktilem Empfinden, vollere Haare zu haben.

Diese kann gemessen werden, indem geschulte Friseurinnen das Volumen im Halbseitentest auf einer Skala von 1 bis 10 beurteilen (Volumenanfühlwert).

Als kationische Tenside können Monoalkylquats (insbesondere Cetrimonium Chloride, Behentrimonium Chloride, Behentrimonium Methosulfate), Esterquats (insbesondere Distearoylethyl Hydroxyethylmonium Methosulfate) oder Amidoamine (insbesondere Stearamidopropyl Dimethylamine) verwendet werden, besonders bevorzugt letztgenannte Amidoamine, ganz besonders bevorzugt Stearamidopropyl Dimethylamine.

Bevorzugt ist es, wenn als kationisches Tensid ein Amidoamin, besonders bevorzugt Stearamidopropyl Dimethylamine verwendet wird. Dadurch kommt es zu einer Verbesserung des Volumens der Frisur bzw. der behandelten Anordnung der keratinischen Fasern. Als weitere Amidoamine können Stearamidopropyldimethylamine, Stearamidopropyldiethylamine, Stearamidoethyldiethylamine, Stearamidoethyldimethylamine, Palmitamidopropyldimethilamine, Palmitamidopropyldiethilamine, Palmitamidoethyldiethilamine, Palmitamidoethyldimethilamine, Behenamidopropyldimethylamine, Behenamidopropyldiethylamine, Behenamidoethyldiethylamine, Behenamidoethyldimethylamine, Arachidamidopropyldimethylamine, Arachidamidopropyldiethylamine, Arachidamidoethyldiethylamine, Arachidamidoethyldimethylamine verwendet werden. Besonders bevorzugt ist es, wenn der Gehalt an Polymer (I) 1 bis 3 Gew.-% beträgt. Dadurch wird eine gute Pflegeperformance erreicht. Weiter bevorzugt ist es, wenn der Gehalt an Polyquaternium-68 5 bis 15 Gew.-% beträgt. Weiter bevorzugt ist es, wenn der pH-Wert der Zubereitung 4 bis 5 beträgt. Dadurch wird ein zusätzliches Zusammenziehen der Haarschuppen bewirkt, was die Kämmbarkeit und Glanz verbessert. Weiter bevorzugt ist es, wenn der Volumenanfühlwert der Zubereitung auf einer Skala von 1-10 (nicht nur taktil, auch visuell) größer als 6,5 ist.

Weiter bevorzugt ist es, wenn die Zubereitung dafür vorgesehen ist 1 bis 1800 s nach der Anwendung von den behandelten Fasern überwiegend abgespült zu werden. Die Zubereitung wirkt auch dann volumenförderd, wenn das Produkt wieder vollständig ausgespült wird. Die Erfindung umfasst auch die Verwendung von erfindungsgemäßen Zubereitungen nach einem der vorangehenden Patentansprüche zur Vergrößerung des Frisurvolumens und die Verwendung von erfindungsgemäßen Zubereitungen wobei das Produkt 1 bis 1800 s nach der Anwendung von den behandelten Fasern überwiegend abgespült wird.

Von Vorteil ist es, wenn auf den Einsatz von Octyl Acrylamide / Acrylates / Butylaminoethyl Methacrylate Copolymer verzichtet werden kann.

Solche Zubereitungen können vorteilhaft kationische Tenside (Alkylamine, Alkylimidazole, ethoxylierte Amine, quaternäre Tenside, Esterquats), Fettalkohole (Cetylalkohol, Cetearylalkohol, Stearylalkohol, Behenylalkohol), Polyole, Cellulosederivate (Hydroxyethylcellulose, Hydroxypropylcellulose, Hydroxymethylcellulose), Pflanzenöle (Sonnenblumenöl, Weizenkeimöl), Parfümöle (Alpha-Isomethyl lonone, Linalool, Limonene, Butylphenyl Methylpropional), Pflanzenextrakte (Bambusa Vulgaris, Vanilla Planifolia, Passiflora Incarnata, Punica Granatum, Nymphaea Odorata), Antioxidantien (Aminosäuren und ihre Derivate, Imidazole und ihre Derivate, Peptide und ihre Derivate, Carotinoide, Carotine und ihre Derivate), Parabene (Propylparaben, Methylparaben) und hydrolisierte Proteine (z. B.: hydrolysed wheat proteine, hydrolysed rice proteine) enthalten.

### Beispiele

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

Die in Tabelle 1 aufgeführten Haarspülungsformeln wurden im Haarstudio an 10 Personen gegen das Handelsprodukt "Pantene Volumen Pur" aus dem Jahr 2007, ein bekanntes Produkt im Segment Spülungen für feines Haar, getestet.

Aus den Ergebnissen zeigt sich ein synergistischer Effekt aus den beiden eingesetzten Polymeren, was den Parameter Volumen betrifft.

Formulierungen, die jeweils nur eines der Polymere enthalten, verursachen weniger Volumen im Haar als die Kombination beider Polymere, selbst in im Wesentlichen der gleichen Summenkonzentration. Dabei konnten maximal 2 Gew.-% PQ-68 verwendet werden, da sich höhere Aktivgehalte schwer in der Formel stabil einarbeiten lassen.

Die Formulierungen wurden jeweils im Halbseitentest gegen das schon genannte Vergleichsprodukt getetstet.

**Tabelle 1: getestete Spülungsformulierungen**

| | inci | 1 | 2 | 3 |
|---|---|---|---|---|
| 1 | Methylparaben | 0,25 | 0,25 | 0,25 |
| 2 | Propylparaben | 0,1 | 0,1 | 0,1 |
| 3 | Aqua | ad 100 | ad 100 | ad 100 |
| 4 | Cetyl Alcohol | 3 | 3,75 | 3,75 |
| 5 | Dimethicone | 3 | 3 | 3 |
| 6 | Lactic Acid | 0,7 | 0,7 | 0,7 |
| 7 | Hydroxyethylcellulose | | 0,25 | 0,25 |
| 8 | Sodium Chloride | 0,01 | 0,01 | 0,01 |
| 9 | Stearyl Alcohol | 5,76 | 7,2 | 7,2 |
| 10 | Oryzanol | 0,05 | 0,05 | 0,05 |
| 11 | Coco Betaine | 0,85 | 0,85 | 0,85 |
| 12 | Polyquaternium-68 | | 7,5 | 10 |
| 13 | Stearamidopropyl Dimethylamine | 1,65 | 1,65 | 1,65 |
| 14 | Parfum | 0,7 | 0,7 | 0,7 |
| 15 | Polymer 1 (Copolymer aus tert. Butylacrylat, Vinylpyrrolidon, Methacrylsäure, Dimethylaminopropylmethacrylamid, Methacryloyloxyethyldimethylethyl Ammonium Ethylsulfat) | 3 | 1,5 | |
| | pH (1d) | 4,2 | 4,7 | 4,9 |
| | Viskosität (1d) | 2150 mPas | 3450 mPas | 3100 mPas |

**Tabelle 2: Rohstoffhersteller und Aktivgehalte**

| Rohstoffe und Aktivgehalte | | | | |
|---|---|---|---|---|
| 1 | Nipagin M, Clariant, 99.5% | | | |
| 2 | Nipasol M, Clariant, 99.5% | | | |
| 4 | Nacol 16-95, Sasol, 95% | | | |
| 5 | Mirasil DM 100, Rhodia, 100% | | | |
| 6 | Purac Hi Pure, Purac, 90% | | | |
| 7 | Tylose H 100000 P2PHA, Shinezu, 99,99% | | | |
| 8 | Cisne Refinado Tarja Verde, Refinaria de sal S.A., 100% | | | |
| 9 | Lanette 18, Cognis, 100% | | | |
| 10 | Gamma Orizanol, Henry Lamotte, 98% | | | |
| 11 | Dehyton AB 30, Cognis, 31% | | | |
| 12 | Luviquat Supreme, BASF, 20% | | | |
| 13 | Tego Amid S 18, Evonik Goldschmidt, 100% | | | |
| 14 | Pf. Highlights, Givaudan | | | |
| 15 | Polymer 1, experimentelles Polymer, BASF | | | |

**Tabelle 3: Ergebnisse Haarstudiotest, 10 Personen, Parameter Haarvolumen**

| | | | | |
|---|---|---|---|---|
| | 1 | 2 | 3 | Benchmark im Bereich Volumen Pflegespülungen |
| Volumen (Skalenbewertung von 0-10) | 7,3 | 7,9 | 7,2 | 6,2 |

**Tabelle 4: weitere Beispielrezepturen**

| | inci | 1 | 2 | 3 | 4 | 5 | 6 | 7 | 8 |
|---|---|---|---|---|---|---|---|---|---|
| 1 | Methylparaben | 0,25 | 0,25 | 0,25 | 0,25 | | 0,25 | 0,25 | 0,25 |
| 2 | Propylparaben | 0,1 | 0,1 | 0,1 | 0,1 | | 0,1 | 0,1 | 0,1 |
| 3 | Glyceryl Stearate | | | | | 1 | | | |
| 4 | Aqua | Ad 100 | Ad 100 | Ad 100 | Ad 100 | Ad 100 | Ad 100 | Ad 100 | Ad 100 |
| 5 | Octyldodecanol | | | | | | 1 | | |
| 6 | Citric Acid | 0,01 | 0,005 | | | 0,025 | | | |
| 7 | Cetearyl Alcohol | 3,1 | | 5,24 | 5,74 | 1 | | | |
| 8 | Cetyl Alcohol | | 2 | | | | 3,81 | 4,05 | 4,05 |
| 9 | Dimethicone | | 1 | | 1 | | 3 | 3 | 3 |
| 10 | Lactic Acid | | | | | | 0,85 | 0,85 | 0,85 |
| 11 | Benzophenone-4 | | 0,25 | 0,25 | 0,25 | | | | |
| 12 | Bis-Diglyceryl Polyacyladipate-2 | 2 | 1 | 1 | 1 | | | | |
| 13 | Glycerin | 8,7 | 0 | 0 | 5 | | | | |
| 14 | Aqua + Sodium Hydroxide (Achtung !! sollte nicht in der VDK erscheinen) | 0,025 | 0,06 | 0,15 | 0,15 | 0,025 | | | |
| 15 | Hydroxyethylcellulose | | | | | 0,75 | 0,25 | 0,25 | 0,25 |
| 16 | Sodium Chloride | | | | | | 0,01 | 0,01 | 0,01 |
| 17 | Hydroxypropyl Methylcellulose | 0,25 | | | | | | | |
| 18 | Cetearyl Alcohol + Behentrimonium Chloride | 0,2 | | | | | | | |
| 19 | Stearyl Alcohol | | 4 | | | | 7,3152 | 7,776 | 7,776 |
| 20 | Cetrimonium Chloride | 4 | | | | 0,4 | | | |
| 21 | Dicaprylyl Ether | | | | | | 1 | | |
| 22 | Distearoylethyl Hydroxyethylmonium Methosulfate + Cetearyl Alcohol | | 2,00 | 2,00 | 2,86 | | | | |
| 23 | Oryzanol | | | | | | 0,05 | 0,05 | 0,05 |
| 24 | DMDM Hydantoin | | | | | 0,4 | | | |
| 25 | Silicone Quaternium-16 + Undeceth-11 + Butyloctanol + Undeceth-5 | | | | | 2 | | | |
| 26 | Cyclomethicone | | 2 | | | | | | |
| 27 | Palmitamidopropyltrimonium Chloride + Propylene Glycol | | 3,00 | 1,66 | | | | | |
| 28 | Coco Betaine | | | | | | 0,85 | 3,22 | 3,22 |
| 29 | Polyquaternium-68 + Phenoxyethanol + Methylparaben + Ethylparaben + Butylparaben + Isobutylparaben + Propylparaben | | | | | | 7,5 | 3,75 | 1,875 |
| 30 | Stearamidopropyl Dimethylamine | | | | | | 2,2 | 2,2 | 2,2 |
| 31 | Parfum | 0,6 | 0,7 | 0,7 | 0,7 | 0,2 | 0,7 | 0,7 | 0,7 |
| 32 | Polymer 1 (Copolymer aus tert. Butylacrylat, Vinylpyrrolidon, Methacrylsäure, Dimethylaminopropylmethacrylamid, Methacryloyloxyethyldimethylethyl Ammonium Ethylsulfat) | 1,5 | 1 | 1,2 | 1,5 | 1 | 1,5 | 0,75 | 0,375 |

**Tabelle 5: Rohstoffe und Aktivgehalte zu Tabelle 4**

| Rohstoffe und Aktivgehalte | |
|---|---|
| 1 | Nipagin M, Clariant, 99.5% |
| 2 | Nipasol M, Clariant, 99.5% |
| 3 | Tegin M Pellets resp. Powder, Evonik Goldschmidt |
| 5 | Isofol 20, Sasol, 100% |
| 6 | Citric Acid monohydrate fine granular 04, DSM Nutritional Products Europe, 100% |
| 7 | Nafol 1618 Ja, Sasol, 100% |
| 8 | Nacol 16-95, Sasol, 95% |
| 9 | Mirasil DM 100, Rhodia, 100% |
| 10 | Purac Hi Pure, Purac, 90% |
| 11 | Escalol 577, ISP, 100% |
| 12 | Softisan 649, Sasol, 100% |
| 13 | Glycerol EP vegetable, Spiga Nord, 99% |
| 14 | Natronlauge 45% techn.rein,Reher & Ramsden, 45% |
| 15 | Tylose H 100000 P2PHA, Shinezu, 99,99% |
| 16 | Cisne Refinado Tarja Verde, Refinaria de sal S.A., 100% |
| 17 | Tylose E707002, SE Tylose, 100% |
| 18 | Incroquat Behenyl TMC-25, Croda, 25% |
| 19 | Lanette 18, Cognis, 100% |
| 20 | CTAC 6025, KCl, 25% |
| 21 | Cetiol OE, Cognis, 96% |
| 22 | Dehyquart 75, Cognis, 69,9% |
| 23 | Gamma Orizanol, Henry Lamotte, 98% |
| 24 | Hydantol 55 KC, KCl, 54% |
| 25 | 5-7113 Silicone Quat Microemulsion, Dow Corning, 22% |
| 26 | Mirasil CM5, Rhodia, 100% |
| 27 | Varisoft PATC, Evonik Goldschmidt, 60% |
| 28 | Dehyton AB 30, Cognis, 31% |
| 29 | Luviquat Supreme, BASF, 20% |
| 30 | Tego Amid S 18, Evonik Goldschmidt, 100% |
| 32 | Polymer 1, experimentelles Polymer, BASF |

## Patentansprüche

1. Kosmetische Zubereitungen zur Behandlung keratinischer Fasern mit kationischen Tensiden, enthaltend Polyquaternium-68 und Polymer (I) zusammengesetzt aus 45 bis 49 Gew.-% tert. Butylacrylat, 23 bis 27 Gew.-% Vinylpyrrolidon, 9 bis 11 Gew.-% Dimethylaminopropylmethacrylamid, 13 bis 17 Gew.-% Methacryloyloxyethyldimethylethyl Ammonium Ethylsulfat, 2 bis 4 Gew.-% Methacrylsäure, besonders bevorzugt aus 47 Gew.-% tert. Butylacrylat, 25 Gew.-% Vinylpyrrolidon, 10 Gew.-% Dimethylaminopropylmethacrylamid, 15 Gew.-% Methacryloyloxyethyldimethylethyl Ammonium Ethylsulfat, 3 Gew.-% Methacrylsäure mit einem Molekulargewicht von 80 000 bis 240 000 g/mol.

2. Zubereitungen nach Patentanspruch 1 **dadurch gekennzeichnet, dass** als kationisches Tensid ein Amidoamin verwendet wird.

3. Zubereitung nach einem der vorangehenden Patentansprüche **dadurch gekennzeichnet, dass** der Gehalt an Polymer (I) 1 bis 3 Gew.-% beträgt.

4. Zubereitung nach einem der vorangehenden Patentansprüche **dadurch gekennzeichnet, dass** der Gehalt an Polyquaternium-68 1 bis 3 Gew.-% bezogen auf den Aktivgehalt beträgt.

5. Zubereitung nach einem der vorangehenden Patentansprüche **dadurch gekennzeichnet, dass** der pH-Wert der Zubereitung 4 bis 5 beträgt.

6. Zubereitung nach einem der vorangehenden Patentansprüche **dadurch gekennzeichnet, dass** der Volumenanfühlwert (nicht nur taktil, auch visuell) der Zubereitung größer als 6,5 ist.

7. Zubereitung nach einem der vorangehenden Patentansprüche **dadurch gekennzeichnet, dass** die Zubereitung dafür vorgesehen ist 1 bis 1800 s nach der Anwendung von den behandelten Fasern überwiegend abgespült zu werden.

8. Verwendung von Zubereitungen nach einem der vorangehenden Patentansprüche zur Vergrößerung des Frisurvolumens.

9. Verwendung nach Patentanspruch 9 wobei das Produkt 1 bis 1800 s nach der Anwendung von den behandelten Fasern überwiegend abgespült wird.
